# FASCICULE DE BREVET EUROPEEN

(11) **EP 3 370 832 B1**
(45) Date de publication et mention de la délivrance du brevet: **28.12.2022**
(21) Numéro de dépôt: 16804829.6
(22) Date de dépôt: 02.11.2016
(51) Int. Cl.: A61K 8/9717, A61K 8/9789, A61Q 17/00, A61Q 19/08

(54) **COMPOSITION COSMÉTIQUE OU DERMATOLOGIQUE QUI COMPREND UN EXTRAIT DE JANIA RUBENS ET UNE HUILE ESSENTIELLE D'IMMORTELLE ET SON UTILISATION**
KOSMETISCHE ODER DERMATOLOGISCHE ZUBEREITUNG ENTHALTEND EINEN EXTRAKT AUS JANIA RUBENS UND EIN ÄTHERISCHES IMMORTELLENÖL UND VERWENDUNGEN DAVON
COSMETIC OR DERMATOLOGICAL COMPOSITION COMPRISING AN EXTRACT OF JANIA RUBENS AND AN ESSENTIAL OIL OF IMMORTELLE AND USES THEREOF

(30) Priorité: 06.11.2015 FR 1560663
(43) Date de publication de la demande: 12.09.2018
(73) Titulaire: Laboratoires M & L, 04100 Manosque (FR)
(72) Inventeur: PORTES, Pascal, 13540 Puyricard (FR); CENIZO, Valérie, 13650 Meyrargues (FR); LEMAIRE, Géraldine, 04220 Corbières-en-Provence (FR)
(74) Mandataire: Novagraaf Technologies
(86) Numéro de dépôt international: PCT/FR2016/052835
(87) Numéro de publication internationale: WO 2017/077232

(56) Documents cités:
- FR-A1- 2 829 145
- FR-A1- 3 000 389
- US-A1- 2009 269 419
- DATABASE GNPD [Online] MINTEL; juin 2015 (2015-06), Delaron: "SlimmingPerfection Cream", XP002757729, Database accession no. 3079483
- "Ultimate Youth Serum", GNPD; MINTEL, septembre 2011 (2011-09), XP002718573,
- "Lifting Radiance Serum", GNPD; MINTEL, octobre 2010 (2010-10), - octobre 2010 (2010-10), XP002715058, [extrait le 2010-10-01]
- SALA A ET AL: "ANTI-INFLAMMATORY AND ANTIOXIDANT PROPERTIES OF HELICHRYSUM ITALICUM", JOURNAL OF PHARMACY AND PHARMACOLOGY, JOHN WILEY & SONS LTD, LONDON; GB, vol. 54, no. 3, 1 mars 2002 (2002-03-01), pages 365-371, XP008025203, ISSN: 0022-3573, DOI: 10.1211/0022357021778600

## Description

### Domaine technique

La présente invention se rapporte à une composition cosmétique comprenant au moins un extrait de Janie rouge, une huile essentielle d'immortelle et au moins un extrait aqueux d'immortelle et/ou un extrait huileux d'immortelle, dans laquelle la concentration d'extrait de Janie rouge est de 0,001% à 5% en poids par rapport au poids total de la composition.

La présente invention se rapporte également à l'utilisation cosmétique d'une composition comprenant au moins un extrait de Janie rouge, une huile essentielle d'immortelle et au moins un extrait aqueux d'immortelle et/ou un extrait huileux d'immortelle, dans laquelle la concentration d'extrait de Janie rouge est de 0,001% à 5% en poids par rapport au poids total de la composition dans un traitement cosmétique non thérapeutique anti-âge.

La présente invention se rapporte également à une composition cosmétique comprenant au moins un extrait de Janie rouge, une huile essentielle d'immortelle et au moins un extrait aqueux d'immortelle et/ou un extrait huileux d'immortelle, dans laquelle la concentration d'extrait de Janie rouge est de 0,001% à 5% en poids par rapport au poids total de la composition pour son utilisation dans un traitement pour la protection de la peau des agressions extérieures.

La présente invention se rapporte également à un procédé de traitement cosmétique non thérapeutique comprenant l'application d'une composition cosmétique comprenant au moins un extrait de Janie rouge, une huile essentielle d'immortelle et au moins un extrait aqueux d'immortelle et/ou un extrait huileux d'immortelle, dans laquelle la concentration d'extrait de Janie rouge est de 0,001% à 5% en poids par rapport au poids total de la composition.

La présente invention se rapporte également à une trousse de soin cosmétique comprenant notamment une composition cosmétique comprenant au moins un extrait de Janie rouge, une huile essentielle d'immortelle et au moins un extrait aqueux d'immortelle et/ou un extrait huileux d'immortelle, dans laquelle la concentration d'extrait de Janie rouge est de 0,001% à 5% en poids par rapport au poids total de la composition.

La présente invention trouve une application notamment dans les domaines cosmétique et/ou dermatologique.

Dans la description ci-dessous, les références entre crochets ([]) renvoient à la liste des références présentée à la fin du texte.

### Etat de la technique

La peau possède une architecture très complexe. Barrière entre les milieux extérieur et intérieur de notre corps, son fonctionnement a deux finalités : assurer la communication entre l'organisme et l'environnement extérieur et le protéger des agressions [1].

Avec l'âge, les différentes structures cutanées sont modifiées à la fois sur le plan morphologique et fonctionnel. Les principales conséquences sont un déclin des fonctions de défense, de cicatrisation, de perception et de thermorégulation ainsi que l'installation d'un état inflammatoire chronique correspondant à une augmentation du nombre de macrophages et d'une sécheresse cutanée persistante [2].

Au niveau de l'épiderme, son renouvellement (turn-over) passe de 21 jours à 40 jours d'où l'impact négatif sur la cicatrisation et la réparation tissulaire [3]. Les kératinocytes voient leur taille diminuer et leur nombre se réduire. Le renouvellement cellulaire se ralentit et la cohésion entre le derme et l'épiderme s'atténue.

Par son renouvellement constant, l'épiderme est la barrière physique contre les agressions chimiques, physiques et bactériennes. Les kératinocytes épidermiques subissent des changements morphologiques et biochimiques lorsqu'ils migrent de la couche basale vers les couches spineuses et granuleuses pour former le stratum corneum. Différentes protéines caractérisent chaque couche de l'épiderme, les kératines 5 et 14 sont les kératines principales de la couche basale alors que les kératines 1, 2 et 10 sont exprimées par les kératinocytes supra-basaux. Certaines protéines qui sont associées à la différenciation telles que l'involucrine, la loricrine et la filaggrine sont uniquement exprimées dans les couches différenciées de l'épiderme [4, 5, 6].

Le processus de cornification est initié dans le *stratum spinosum* avec l'expression des protéines involucrine [7], periplakine [8] et envoplakine [9]. Initialement, ces 2 membres de la famille des plakines (periplakine et envoplakine) forment un complexe avec l'involucrine et sont attachées à la surface intérieure de la membrane plasmique [10], formant un assemblage [11]. Le recrutement ainsi que la liaison de la periplakine et de l'envoplakine à la bicouche lipidique requiert la présence de calcium. Ces 3 protéines (envoplakine, involucrine et periplakine) se retrouvent réticulées par la transglutaminase 1 dont l'activité enzymatique dépend du calcium. La formation en continue de l'enveloppe cornée requiert la production des corps lamellaires dans le stratum granulosum. La sécrétion du contenu des corps lamellaires apporte à la peau son imperméabilité et la protège des bactéries.

Le composant principal de l'enveloppe cornée est la loricrine. Cette protéine représente plus de 80% de la masse des protéines de l'enveloppe cornée et est exprimée dans le stratum granulosum [12]. La synthèse de la loricrine requiert une concentration en calcium d'environ 0,1 mM. Durant le processus de formation de l'enveloppe cornée, la loricrine est réticulée à elle-même et aux membres de la famille des SPRR (small proline rich repeat) [13, 14]. L'addition de calcium aux kératinocytes en culture primaire augmente fortement l'expression des SPRRs. Elles sont ainsi réticulées à la loricrine via les ponts Nε-(γ glutamyl) lysine (isopeptide) par les transglutaminases 1 et 3 [13, 15]. Durant le processus final de formation de l'enveloppe cornée, l'assemblage loricrine-SPRR est transporté à la membrane cellulaire et attaché à l'assemblage periplakine-involucrine-evoplakine [12]. Les dernières protéines incorporées à l'enveloppe cornée sont les membres de la famille des "late cornified envelope" (LCE) [16, 17].

Au sein de l'épiderme, les kératinocytes ont des besoins différents en calcium. Les kératinocytes de la couche basale ont besoin de concentrations très faibles en calcium permettant la division de cellules souches qui assurent le renouvellement épidermique. Alors que de fortes concentrations en calcium sont nécessaires pour le programme de différenciation résultant en la formation des cornéocytes et de l'enveloppe cornée. Pour que les kératinocytes rencontrent ces différentes concentrations, un gradient de calcium est construit au sein de l'épiderme. Chez les sujets âgés, le gradient en calcium est fortement perturbé. En effet, chez les sujets jeunes ou adultes jeunes un pic de calcium est clairement observé dans la couche supérieure du *stratum granulosum,* alors que chez les sujets âgés, le calcium est distribué de manière homogène dans toutes les couches de l'épiderme [18].

La filaggrine est produite par les kératinocytes granuleux sous la forme d'un précurseur appelé profilaggrine qui est constitué de 10-12 unités de filaggrines. C'est le composant majeur des granules de keratohyaline. Lors de la transition des granules à l'enveloppe cornée, la profilaggrine est rapidement déphosphorylée et clivée par certaines protéases telles que la caspase-14, la bléomycine hydrolase, et la calpaine 1 [19, 20, 21] pour générer les monomères de filaggrine [21]. Ces monomères de filaggrine vont s'associer avec des filaments de kératine et sont suspectés d'induire la formation de la matrice fibreuse des cornéocytes [22]. Dans les cellules cornées, la filaggrine est dégradée en acides aminés libres et d'autres composants du facteur naturel d'hydratation de la peau. Ces acides aminés libres sont nécessaires pour la photo-protection de la peau (acide urocanique), ainsi que l'acidification et l'hydratation (pyrrolidone carboxylic acid) du stratum corneum [21, 23, 24].

Avec l'âge, le renouvellement des kératinocytes est considérablement ralenti et la composition protéique de l'enveloppe cornée responsable de la fonction barrière est profondément modifiée. Il est ainsi important de restaurer la prolifération des kératinocytes ainsi que leur potentiel de différenciation dans le but de restaurer l'homéostasie épidermique et la fonction barrière de la peau qui sont perturbées par le vieillissement de la peau.

Par ailleurs, il est connu qu'avec l'âge la peau perd de sa fermeté et de son élasticité qui peut être notamment à l'origine de l'apparition de rides tant au niveau du visage que sur l'ensemble du corps.

Il existe dans l'état de la technique de nombreuses compositions cosmétiques pour le traitement du vieillissement cutané et/ou raffermir la peau. Toutefois, ces compositions présentent des efficacités relatives, voire contestées dans certains cas par des associations de consommateurs. L'efficacité relative des compositions connues peuvent être à l'origine d'un besoin pour les individus de recourir à des actes chirurgicaux tels que l'injection d'acide hyaluronique et/ou de toxine botulique afin de masquer les rides.

Un extrait aqueux ou hydroglycérique de Jania rubens est connu comme pouvant stimuler la synthèse des fibres de collagène, inhiber la différenciation des adipocytes et stimuler la lipolyse (Brevet FR3000389) [25]. L'huile essentielle d'immortelle est également connue pour avoir des propriétés anti-radicalaires, stimuler la synthèse des fibres de collagène et stimuler l'expression de la sirtuine 1 et/ou induire l'expression génique de la loricrine et de deux « S100 calcium binding protein » (S100A7 et S100A9) qui sont des protéines impliquées dans la différenciation épidermique [26, 27, 28, 29]. Le document Database GNPD mintel; Delaron: « Slimming Perfection Cream » juin 2015, AN : 3079483 décrit une composition cosmétique comprenant notamment un extrait de café vert, un mélange d'huiles essentielles et un extrait de Jania rubens. En particulier, ce document enseigne que la composition est une crème amincissante pour l'abdomen, les hanches et les cuisses (...) éliminant les toxines (...) cellulites etc..

Le document Database GNPD mintel; « ultimate youth sérum", septembre 2011 décrit une composition cosmétique comprenant notamment une huile essentielle d'immortelle et une huile essentielle de myrte (page 1, 1^{er} paragraphe). Ce document décrit également que la composition est une composition anti-âge dans laquelle l'huile essentielle d'immortelle stimule la production de collagène afin de lutter contre les rides, la fermeté de la peau, la protéger des radicaux libres, et augmenter sa luminosité (page 1, 1^{er} paragraphe).

Le document Database GNPD mintel « lifting radiance Serum », octobre 2010 décrit une composition comprenant notamment un extrait de Jania Rubens ("ingredients"). Ce document décrit également que le composé Jania Rubens est un ingrédient clé (« Key ingrédient ») avec un effet ultra hydratant. En particulier, ce document décrit également que Jania Rubens est un hydratant et que la composition décrite comprend des pigments réflecteurs de lumière.

Toutefois, ces compositions ne permettent a priori pas de traiter efficacement à la fois le vieillissement intrinsèque et extrinsèque de la peau, les causes de ce vieillissement cutané tout en protégeant la peau des agressions extérieures. En d'autres termes, les compositions cosmétiques connues présentent des effets relatifs pour lesquels il existe un réel besoin des compositions et/ou moyens permettant de traiter le vieillissement intrinsèque et extrinsèque de la peau, les causes de ce vieillissement cutané tout en protégeant la peau des agressions extérieures.

Il existe également dans l'état de la technique de nombreuses compositions cosmétiques pour le traitement du vieillissement cutané et/ou raffermir la peau. Toutefois, ces compositions comme celles mentionnées ci-dessus présentent des efficacités relatives.

Il existe donc un réel besoin de trouver des compositions cosmétiques anti-âge permettant notamment de renforcer la barrière cutanée, lisser la surface de la peau, raffermir la peau et/ou prévenir ou traiter le vieillissement cutané, par exemple le vieillissement cutané intrinsèque et/ou extrinsèque.

### Description de l'invention

La présente invention a précisément pour but de répondre à ces besoins et inconvénients en fournissant une composition cosmétique comprenant au moins un extrait de Janie rouge, une huile essentielle d'immortelle et au moins un extrait aqueux d'immortelle et/ou un extrait huileux d'immortelle, dans laquelle la concentration d'extrait de Janie rouge est de 0,001% à 5% en poids par rapport au poids total de la composition.

Les inventeurs de la présente invention sont en effet les tout premiers à avoir mis en évidence, de manière tout à fait inattendue, qu'une composition cosmétique comprenant à la fois au moins un extrait de Janie rouge et au moins un extrait d'immortelle permet avantageusement un effet anti-âge, notamment via la stimulation de la prolifération et de la différenciation des cellules épidermiques, tout en protégeant des agressions extérieures de par le renforcement de la fonction barrière de la peau.

En outre, les inventeurs de la présente invention ont également démontré de manière surprenante et inattendue, qu'une composition cosmétique comprenant à la fois au moins un extrait de Janie rouge et au moins un extrait d'immortelle permet avantageusement de conserver l'hydratation de la peau tout en stimulant le renouvellement épidermique.

Dans la présente par « extrait » on entend toute forme d'extrait connu de l'homme du métier. Il peut s'agir par exemple d'un extrait liposoluble, d'un extrait hydrosoluble, d'un extrait huileux, d'un extrait aqueux, d'un extrait-hydro-alcoolique, d'un extrait hydro-glycériné, d'un extrait supercritique, d'une huile essentielle ou un quelconque mélange de ceux-ci.

Dans la présente l'extrait peut être obtenu par exemple à partir de tout ou partie d'une plante. Il peut s'agir par exemple d'un extrait obtenu à partir de fleurs ou sommités fleuries, feuilles, graines, racines, tiges, péricarpe. Il peut s'agir par exemple d'un extrait obtenu à partir de fleurs entières.

Dans la présente par « extrait liposoluble » on entend un extrait riche en acide gras et/ou en matière grasse et/ou un extrait non hydrosoluble. Selon l'invention, l'extrait huileux peut être tout extrait huileux obtenu par tout procédé connu de l'homme du métier. Par exemple, il peut être obtenu par extraction assistée par hyperfréquences ou par ultrasons, par extraction supercritique, par macération dans un solvant huileux [30]

Dans la présente par « extrait hydrosoluble » on entend un extrait soluble dans l'eau. Il peut s'agir d'un extrait aqueux, hydro-alcoolique, hydro-glycériné; obtenu par distillation, par macération, par extraction assistée, par extraction subcritique, par hyperfréquences ou par ultrasons [30]

Dans la présente par « immortelle » on entend une espèce de plantes à fleurs de la famille des Asteraceae et du genre Helichrysum. En particulier, il peut s'agir de l'*Helichrysum italicum* qui peut être présente tout autour de la Méditerranée. Il peut s'agir par exemple de l'immortelle décrite dans les brevets Français FR0111224, FR0605953 et FR0905904 [26, 27, 28].

« L'extrait d'immortelle» décrit peut être un extrait liposoluble, un extrait hydrosoluble, une huile essentielle ou un mélange de ceux-ci.

Selon l'invention, l'extrait d'immortelle comprend une huile essentielle d'immortelle et un extrait aqueux d'immortelle, et/ou un extrait huileux d'immortelle. Dans la présente, l'extrait liposoluble peut être obtenu à partir d'une plante et/ou d'une partie d'une plante. Il peut s'agir par exemple d'un extrait huileux obtenus à partir de fleurs ou sommités fleuries, feuilles, graines, racines, tiges, péricarpes.

Il peut s'agir par exemple d'un extrait liposoluble d'immortelle obtenu par tout procédé connu de l'homme du métier. Il peut s'agir par exemple d'un extrait huileux riche en acide gras et/ou en matière grasse obtenu par tout procédé connu de l'homme du métier. Par exemple, il peut s'agir d'un extrait huileux obtenu par extraction huileuse par micro-ondes, par hyperfréquence, par extraction supercritique, par macération dans un solvant huileux, par extraction huileuse assistée par ultra-sons. Il peut s'agir par exemple d'un extrait huileux biologique d'immortelle par extraction micro-ondes assistée par ultra-sons selon un procédé comprenant une première étape de broyage de la sommité fleurie d'immortelle suivie d'une extraction assistée par ultrasons et par micro-ondes, puis une clarification et enfin une étape de filtration afin de récupérer l'extrait huileux.

Dans la présente, l'extrait liposoluble peut être un extrait huileux et/ou une huile essentielle.

Dans la présente, l'huile essentielle peut être obtenue, par exemple d'une huile obtenue à partir de fleurs ou sommités fleuries, feuilles, graines, racines, tiges. Il peut s'agir par exemple d'une huile essentielle d'immortelle obtenue à partir des sommités fleuries. Il peut s'agir par exemple d'une huile essentielle biologique d'immortelle, c'est-à-dire issue de l'agriculture biologique, par exemple une huile essentielle biologique de Corse, par exemple des sociétés TEPPE ROSSE, STEPHAN FRANCISCI, GAEC de l'ASTRATELLA ou mélange de deux ou trois de ces huiles.

Dans la présente, l'huile essentielle biologique d'immortelle peut comprendre de 25 à 50 % en poids d'acétate de néryle et/ou de 2 à 15% d'italidiones. Par exemple l'huile essentielle biologique d'immortelle peut comprendre de 25 à 50 % en poids d'acétate de néryle et/ou de 2 à 10% d'italidiones.

Dans la présente l'extrait huileux d'immortelle peut comprendre de 0,1 à 0,3% en poids de sucres, par exemple 0,2% de sucres, de 0,04 à 0,06% en poids de vitamine E, par exemple 0,051% de vitamine E, de 99% à 99,9% en poids d'acides gras, par exemple 99,8% en poids d'acides gras, dont de 80 à 85% d'acide oléique, par exemple 83,4% d'acide oléique, de 6 à 8% d'acide linoléique (oméga-6), par exemple 7,3% d'acide linoléique (oméga-6), de 2 à 5% d'acide palmitique et de 2 à 4% d'acide stéarique mais se distingue surtout par sa teneur en phytostérols de 0,2% à 0,5%, par exemple 0,35% dont une majorité de béta-sitostérol 0,2% et en polyphénols de 0,01 à 0,1%, par exemple 0,074%.

Dans la présente un extrait CO₂ supercritique est décrit, par exemple obtenu à partir de fleurs ou sommités fleuries, feuilles, graines, racines, tiges. Il peut s'agir par exemple d'un extrait CO₂ supercritique obtenu à partir de parties aériennes sèches ou sommités fleuries sèches. Il peut s'agir par exemple d'un extrait obtenu à partir de parties aériennes sèches par la mise en œuvre du procédé d'extraction par fluides supercritiques, par exemple C0₂ supercritique [31].

Dans la présente, l'extrait hydrosoluble d'immortelle peut, par exemple, être un extrait hydrosoluble d'immortelle obtenu par tout procédé connu de l'homme du métier. Il peut s'agir d'un extrait aqueux obtenu selon le procédé décrit dans l'ouvrage « Eco-extraction du végétal » [30]. Il peut s'agir, par exemple d'un procédé d'extraction dit « éco-procédé » c'est-à-dire consommant moins d'énergie que les procédés traditionnels. Il peut s'agir par exemple d'un procédé d'extraction assistée par ultrasons. Il peut s'agir par exemple d'un procédé d'extraction à l'eau subcritique obtenu selon le procédé décrit dans l'ouvrage « Eco-extraction du végétal » [30]. Il peut s'agir par exemple d'un extrait aqueux biologique Immortelle, par exemple un extrait d'immortelle biologique de Corse de la société Greentech.

Dans la présente, l'extrait aqueux peut être obtenu par exemple à partir d'une plante et/ou d'une partie d'une plante. Il peut s'agir par exemple d'un extrait aqueux obtenu à partir de fleurs ou sommités fleuries, feuilles, graines, racines, tiges. Il peut s'agir par exemple d'un extrait obtenu à partir de fleurs entières.

Selon l'invention, l'extrait d'immortelle peut être présent dans la composition par exemple à une concentration de 0,01 à 10% en poids par rapport au poids total de ladite composition, par exemple de 0,01 à 5% en poids par rapport au poids total de ladite composition.

Selon l'invention, l'extrait hydrosoluble d'immortelle peut être présent dans la composition par exemple à une concentration de 0,01 à 10% en poids par rapport au poids total de ladite composition, par exemple de 0,01 à 5% en poids par rapport au poids total de ladite composition.

Selon l'invention, l'extrait liposoluble d'immortelle peut être présent dans la composition par exemple à une concentration de 0,001 à 10%, de 0,001 à 5% en poids par rapport au poids total de ladite composition, par exemple de 0,001 à 2% en poids par rapport au poids total de la dite composition.

Selon l'invention, l'huile essentielle d'immortelle peut être présente dans la composition de 0,001% à 10%, de 0,001 à 5% en poids par rapport au poids total de ladite composition, par exemple de 0,001 à 0,5% en poids par rapport au poids total de ladite composition.

Selon l'invention, lorsque la composition comprend un extrait liposoluble d'immortelle, hydrosoluble d'immortelle et de l'huile essentielle d'immortelle la somme des extraits liposoluble d'immortelle, hydrosoluble d'immortelle et de l'huile essentielle d'immortelle dans la composition de l'invention peut être de 0,001 à 10% en poids par rapport au poids total de ladite composition, par exemple de 0,001 à 5% en poids par rapport au poids total de ladite composition.

Dans la présente par «Janie rouge» on entend une algue appartenant à la famille des corallinacées, par exemple se trouvant dans l'Atlantique Nord-Est, la Méditerranée, la mer Noire, de l'océan Indien, la mer de Chine. Il peut s'agir de l'algue *Jania rubens* qui est une algue rouge (Rhodophycées) appartenant aux corallinacées. Cette algue est une espèce calcifiée, constituée de touffes dressées sur une croûte basale commune, et peut mesurer environ 2 à 3 cm de haut. Au cours de sa lente croissance, *Jania rubens* est capable de fixer les minéraux et les oligo-éléments de l'eau de mer mais retient également la microflore et la microfaune marines.

Dans la présente par extrait de Janie rouge on entend un extrait de tout ou partie de Janie rouge. Il peut s'agir par exemple d'un extrait de Janie rouge sauvage et/ou de Janie rouge de culture.

Dans la présente, la Janie rouge peut être l'algue *Jania Rubens.*

Dans la présente l'extrait de Janie rouge peut être un extrait hydrosoluble ou un extrait liposoluble ou un mélange de ceux-ci.

Dans la présente, l'extrait liposoluble de Janie rouge peut être obtenu à partir d'une plante et/ou d'une partie d'une plante.

Dans la présente, l'extrait liposoluble de Janie rouge peut être un extrait huileux, un extrait CO₂ supercritique ou un mélange de ceux-ci.

Dans la présente, l'extrait liposoluble de Janie rouge peut, par exemple, être un extrait huileux de Janie rouge obtenu par tout procédé connu de l'homme du métier. Par exemple, il peut être obtenu par extraction assistée par hyperfréquences ou par ultrasons, par extraction supercritique, par macération dans un solvant huileux selon le procédé décrit dans l'ouvrage [30].

L'extrait liposoluble de Janie rouge peut être par exemple un extrait huileux riche en acide gras et/ou en matière grasse obtenu par tout procédé connu de l'homme du métier. Par exemple, il peut s'agir d'un extrait huileux obtenu par extraction huileuse par micro-ondes, par hyperfréquence, par extraction supercritique, par macération dans un solvant huileux, par extraction huileuse assistée par ultra-sons. Il peut s'agir par exemple d'un extrait huileux biologique de Janie rouge par extraction micro-ondes assistée par ultra-sons selon un procédé comprenant une première étape de broyage suivie d'une extraction assistée par ultrasons et par micro-ondes, puis une clarification et enfin une étape de filtration afin de récupérer l'extrait huileux.

Dans la présente, l'extrait de Janie rouge peut, par exemple, être un extrait hydrosoluble de Janie rouge obtenu par tout procédé connu de l'homme du métier. Il peut s'agir par exemple d'un extrait aqueux de Janie rouge obtenu selon le procédé décrit dans l'ouvrage « Eco-extraction du végétal » [30]. Il peut s'agir, par exemple d'un procédé d'extraction dit « éco-procédé » c'est-à-dire consommant moins d'énergie que les procédés traditionnels. Il peut s'agir par exemple d'un procédé d'extraction assistée par ultrasons. Il peut s'agir par exemple d'un procédé d'extraction à l'eau subcritique tel que décrit dans l'ouvrage « Eco-extraction du végétal » [30] Il peut s'agir par exemple d'un procédé d'extraction hydro-alcoolique. Il peut s'agir par exemple d'un procédé d'extraction hydro-glycérique.

Dans la présente, l'extrait hydrosoluble de Janie rouge peut, par exemple, être un extrait aqueux de Janie rouge obtenu par tout procédé connu de l'homme du métier. Il peut s'agir par exemple d'un extrait aqueux de Janie rouge disponible dans le commerce, par exemple il peut s'agir d'un extrait aqueux commercialisé par la société CODIF.

Dans la présente, l'extrait de Janie rouge peut être, par exemple, un extrait hydroglycérique (50/50 v/v) de Janie rouge. Il peut s'agir par exemple d'un extrait hydroglycérique de *Jania Rubens,* par exemple un extrait hydroglycérique disponible dans le commerce, par exemple commercialisé par la société CODIF sous la référence commerciale Actiporine 8G.

Selon l'invention, l'extrait de Janie rouge peut être présent dans la composition à une concentration de 0,001 à 5% en poids par rapport au poids total de ladite composition.

Selon l'invention, l'extrait hydrosoluble de Janie rouge peut être présent dans la composition à une concentration de 0,01 à 5 % en poids par rapport au poids total de ladite composition.

Selon l'invention, l'extrait hydroglycérique de Janie rouge peut être présent dans la composition à une concentration de 0,01 à 5% en poids par rapport au poids total de la dite composition.

Selon l'invention, l'extrait liposoluble de Janie rouge peut être présent dans la composition à une concentration de 0.001% à 5% en poids par rapport au poids total de la dite composition.

Selon l'invention, lorsque la composition comprend un extrait hydrosoluble de Janie rouge et un extrait liposoluble de Janie rouge, la somme des concentrations des extraits hydrosoluble de Janie rouge et liposoluble de Janie rouge peut être de 0,001 à 5% en poids par rapport au poids total de ladite composition.

Avantageusement, la composition selon l'invention comprend au moins un extrait hydroglycérique de Janie rouge et au moins une huile essentielle d'immortelle.

Avantageusement, les inventeurs ont démontré qu'une composition comprenant au moins un extrait de Janie rouge, par exemple un extrait hydroglycérique de Janie rouge et au moins un extrait d'immortelle, par exemple de l'huile essentielle d'immortelle, permet de stimuler la différenciation épidermique et de renforcer la fonction barrière de la peau. En outre, la composition de l'invention permet avantageusement de favoriser la cohésion entre le derme et l'épiderme, de régénérer et restructurer l'épiderme pour un meilleur lissage en surface et un teint éclatant.

Selon l'invention, quel que soit l'extrait ou le mélange d'extraits utilisés, la composition cosmétique de la présente invention peut comprendre en outre au moins un agent actif additionnel choisi, par exemple parmi un agent anti-âge, un agent hydratant, un agent éclaircissant, un agent apaisant, un agent agissant sur la microcirculation, un ou plusieurs agents anti-oxydants, un agent raffermissant, un agent tenseur ou un mélange de ces agents. Ces agents peuvent être ceux couramment utilisés dans les produits cosmétiques. Des exemples d'agents anti-âges utilisables sont par exemple le silicium ou la vitamine C. Des exemples d'agents hydratants utilisables sont par exemple la glycérine ou l'acide hyaluronique. Des exemples d'agents éclaircissants utilisables sont par exemple l'arbutine ou la vitamine C. Des exemples d'agents apaisants utilisables sont par exemple le bisabolol ou l'allantoïne. Des exemples d'agents agissant sur la micro-circulation utilisables sont par exemple l'escine ou l'hespéritine d'orange. Des exemples d'agents anti-oxydants utilisables sont par exemple le Pycnogenol (marque déposée), la Vitamine C, les polyphénols. Des exemples d'agents raffermissants utilisables sont par exemple des dérivés de seigle. Des exemples d'agents tenseurs utilisables sont par exemple des exopolysaccharides. Ce ou ces agent(s) peuvent être utilisé(s) dans la composition de la présente invention par exemple aux concentrations usuellement utilisées et connues de l'homme du métier dans les compositions cosmétiques ou dermatologiques.

La composition cosmétique selon l'invention peut comprendre en outre au moins un agent actif additionnel choisi, par exemple parmi un agent conditionneur, un agent réparateur. Ces agents peuvent être ceux couramment utilisés dans les produits cosmétiques et/ou dermatologiques. Des exemples d'agents conditionneurs utilisables peuvent être par exemple, des polysaccharides, des protéines hydrolysées. Des agents réparateurs peuvent être par exemple des huiles végétales de tournesol, d'onagre, du beurre de karité.

La composition cosmétique selon l'invention peut comprendre un ou plusieurs adjuvants connus de l'homme du métier. Il peut s'agir par exemple d'un ou plusieurs adjuvant(s) choisi(s) parmi des agents de type esters, des agents hydratants, des agents émollients, des émulsionnants, des tensio-actifs, des agents épaississants minéraux, des agents épaississants organiques, associatifs ou non, des filtres solaires organiques hydrosolubles et liposolubles, des filtres solaires minéraux, des composés siliconés, des parfums, des conservateurs, des céramides, et pseudo-céramides, des vitamines et les provitamines, des protéines, des agents séquestrants, des agents alcalinisants, des agents acidifiants, des agents réducteurs, des agents oxydants, des charges minérales, des colorants ou tout autre adjuvant pouvant être cité dans le dictionnaire INCI (International Nomenclature of Cosmetic Ingredients) publié par le PCPC (Personal Care Products council).

Selon l'invention, la composition cosmétique ou dermatologique peut, par exemple, se présenter sous toute forme connue de l'homme du métier susceptible d'être utilisée dans le domaine cosmétique, sans aucune restriction galénique particulière. La composition peut être sous toute forme galénique connue de l'homme du métier, Il peut s'agir, par exemple d'une émulsion huile-dans-l'eau, eau-dans-l'huile, d'une émulsion multiple, d'une microémulsion, d'une nano-émulsion, d'une émulsion solide, d'un gel aqueux ou hydro-alcoolique, d'une brume, d'un onguent, d'un masque, d'un shampooing, d'un après-shampooing, d'un sérum, d'une lotion capillaire, d'un onguent, d'une crème, d'une huile, d'un lait, d'une pommade, d'un stick, d'un tampon imbibé, d'un patch transdermique, d'une solution, d'un gel, d'un spray, d'une lotion ou d'une suspension, d'une cire, d'un sérum biphasé, par exemple comprenant une fraction huileuse et une fraction aqueuse.

Selon l'invention, la composition cosmétique ou dermatologique peut être une composition topique. Il peut s'agir de toute composition topique connue de l'homme du métier. Il peut s'agir par exemple d'une crème, un sérum, un lait, un gel, une lotion, un onguent, une pommade, une huile, un baume, un masque.

Dans la présente, la composition cosmétique peut être obtenue par tout procédé approprié connu de l'homme du métier pour la fabrication d'une composition cosmétique. Il peut s'agir d'un mélange de matières tensioactives dans de l'eau. Il peut s'agir également, par exemple, d'un procédé comprenant une étape d'incorporation d'une phase interne dans une phase externe au moyen d'un émulseur, par exemple d'une turbine de type rotor-stator. Il peut s'agir également par exemple d'un procédé utilisant la Température d'Inversion de Phase (TIP), procédé classiquement utilisé par l'homme de l'art pour obtenir des émulsions huile dans eau dont les gouttelettes dispersées sont particulièrement fines, par exemple avec un diamètre de 0,1 à 1 µm.

Les inventeurs ont démontré de manière surprenante et inattendue que la composition comprenant un extrait de Janie rouge et un extrait d'immortelle selon l'invention permet avantageusement notamment de part une augmentation de la différenciation des cellules épidermiques, de renforcer la fonction barrière de la peau, de maintenir une meilleure hydratation, de stimuler les défenses de la peau et d'améliorer la qualité de surface de la peau.

Aussi selon l'invention la composition peut être en outre une composition utilisée dans un traitement cosmétique non thérapeutique anti-âge.

Les inventeurs ont également démontré de manière surprenante et inattendue que la composition comprenant un extrait de Janie rouge et un extrait d'immortelle selon l'invention permet avantageusement de maintenir/augmenter l'hydratation de la peau, de prévenir et/ou traiter le vieillissement de la peau, par exemple en conservant et/ou restaurant l'épaisseur de l'épiderme et/ou en améliorant la qualité de surface de la peau.

Les inventeurs ont en outre démontré de manière surprenante et inattendue que la composition comprenant un extrait de Janie rouge et un extrait d'immortelle selon l'invention permet avantageusement de réduire la densité des taches pigmentaires dues au vieillissement cutané et améliore l'uniformité et la clarté du teint.

Selon l'invention, la composition peut être en outre une composition utilisée dans un traitement cosmétique non thérapeutique du vieillissement cutané, des rides et ridules, de la perte de fermeté, de la perte d'élasticité et des peaux sèches.

Selon l'invention, la composition peut être une composition utilisée dans un traitement cosmétique non thérapeutique anti-âge, par exemple dans le traitement du vieillissement cutané, par exemple des rides et ridules, de la perte de fermeté, de la perte d'éclat, de la perte d'élasticité et des peaux sèches.

Les inventeurs ont également démontré de manière surprenante et inattendue que la composition comprenant un extrait de Janie rouge et un extrait d'immortelle permet avantageusement de protéger la peau des agressions extérieures.

Dans la présente par « agressions extérieures » on entend, par exemple d'ordre physique, par exemple par le froid, les micro-organismes, les poussières, le soleil et/ou les Ultraviolets (UV), la pollution, ou une agression chimique, par exemple le tabac, les gaz des pots d'échappements, les particules fines.

Aussi, la présente invention a également pour objet une composition cosmétique comprenant au moins un extrait de Janie rouge, une huile essentielle d'immortelle et au moins un extrait aqueux d'immortelle et/ou un extrait huileux d'immortelle, dans laquelle la concentration d'extrait de Janie rouge est de 0,001% à 5% en poids par rapport au poids total de la composition pour son utilisation dans un traitement pour la protection de la peau des agressions extérieures.

Dans cette application, l'huile essentielle d'immortelle, l'extrait aqueux d'immortelle et l'extrait huileux d'immortelle sont tels que définis ci-dessus

Dans cette application l'extrait de Janie rouge est tel que défini ci-dessus.

Dans cette application la composition est telle que définie ci-dessus.

La présente invention a également pour objet un procédé de traitement cosmétique non thérapeutique comprenant l'application sur la peau d'une composition cosmétique comprenant au moins un extrait de Janie rouge, une huile essentielle d'immortelle et au moins un extrait aqueux d'immortelle et/ou un extrait huileux d'immortelle, dans laquelle la concentration d'extrait de Janie rouge est de 0,001% à 5% en poids par rapport au poids total de la composition.

L'extrait de Janie rouge est tel que défini ci-dessus.

L'huile essentielle d'immortelle, l'extrait aqueux d'immortelle et l'extrait huileux d'immortelle sont tels que définis ci-dessus

La composition cosmétique est telle que définie ci-dessus.

Pour cette application, on peut, par exemple, utiliser les formes galéniques décrites ci-dessus. L'application sur la peau peut donc être réalisée en fonction de la forme galénique utilisée.

Il peut s'agir, par exemple d'une simple application sur la peau ou d'une application accompagnée d'un massage de la peau avec la composition de la présente invention.

L'application est de préférence réalisée avec une quantité suffisante de la composition afin que toute la surface de la peau à traiter soit traitée. Il peut s'agir par exemple d'une application classique, comme une crème sur la peau. Il peut s'agir par exemple aussi d'une application pour former un masque traitant.

L'application peut être, par exemple une application quotidienne, hebdomadaire ou bi-mensuelle. Il peut s'agir par exemple d'une application une fois par jour, deux fois par jour ou plus.

La présente invention a également pour objet une trousse de soin cosmétique comprenant une composition cosmétique selon l'invention et un support comportant des instructions pour l'emploi de la composition.

Le support comportant des instructions pour l'emploi de la composition peut être un manuel ou une notice d'utilisation, notamment pour expliquer à l'utilisateur la manière et la fréquence d'application sur la peau de la composition de la présente invention.

Selon l'invention, la trousse de soin cosmétique peut également comprendre un applicateur, par exemple une spatule, une brosse, un applicateur dynamique, par exemple un dispositif de massage imprégné de la composition cosmétique de l'invention.

D'autres caractéristiques et avantages apparaîtront encore à l'homme du métier à la lecture des exemples ci-dessous, donnés à titre illustratif et non limitatif.

### EXEMPLES

### Exemple 1 : Exemple de composition pour le soin du visage (crème)

Une composition de base a été préparée selon le procédé suivant : les différents ingrédients ont été préalablement préparés et pesés, la glycérine a été introduite dans l'eau et mélangée à 1500 t/min et chauffée à 80°C, le gélifiant a été dispersé dans ce mélange sous agitation à 1500 t/min jusqu'à dissolution. La phase huileuse préalablement préparée par mélange, par exemple de l'émulsionnant, des corps gras durs, des caprylic capric triglycérides, de l'ester et des huiles végétales, à chaud, à 80°C, jusqu'à obtention d'un mélange homogène, a été introduite sous agitation à une vitesse de 1500 tours par minute jusqu'à l'obtention d'une crème qui a été ensuite refroidie à 40°C sous agitation à 1500 t/min. Les actifs, par exemple, les oligo-éléments, le dérivé de vitamine C, ainsi que le parfum, les conservateurs et le régulateur de pH ont été ensuite ajoutés.

Le tableau 1 ci-dessous résume la composition de la crème.

**Tableau 1: Composition de base pour le visage (crème)**

| **Ingrédients** | **Quantité en % de poids par rapport au poids total de la composition** |
|---|---|
| Emulsionnant | 1 à 10% |
| Agents hydratants (glycérine) | 1 à 15% |
| Corps gras durs | 1 à 5% |
| Caprylic capric triglycerides | 5 à 25% |
| Ester | 10-20% |
| Huiles végétales | 1-5% |
| Gélifiant | 1-5% |
| Parfum | QSP |
| Eau | QSP |
| Conservateurs | QSP |

A partir de cette composition de base, pour le soin du visage, ont été fabriquées différentes compositions conformes à la présente invention en ajoutant, un extrait de Janie rouge et un extrait d'immortelle en différentes quantités.

L'extrait de Janie rouge était un extrait hydroglycérique (50/50 v/v) de *Jania rubens* commercialisé par la société CODIF sous la référence commerciale Actiporine 8G.

L'extrait d'immortelle était une huile essentielle d'immortelle obtenue par hydrodistillation et fournie par les sociétés TEPPE ROSSE, STEPHAN FRANCISCI, GAEC de l'ASTRATELLA ou mélange de deux ou trois de ces huiles.

L'extrait d'immortelle était un extrait aqueux d'Immortelle biologique de Corse obtenu par extraction et fournie par la société Greentech.

L'extrait d'immortelle était un extrait huileux obtenu par extraction huileuse par micro-onde assistée par ultra-sons et fournie par la société Oléos.

Les différents ingrédients ont été dispersés à l'aide d'un mélangeur de laboratoire comme un agitateur de paillasse.

A partir de la composition de base, des compositions ont été préparés comprenant :
- 0,1; 0,05; 0,03; 0,02; 0,01% en poids d'extrait huileux d'immortelle par rapport au poids total de chaque composition
- 0,5; 0,2; 0,1 ; 0,05; 0,02; 0,01% en poids d'extrait aqueux d'immortelle par rapport au poids total de chaque composition
- 0,2; 0,1; 0,05; 0,02; 0,01% en poids d'huile essentielle d'Immortelle par rapport au poids total de chaque composition
- 3; 2 ; 1 ; 0,5 ; 0,1 % en poids de Janie rouge par rapport au poids total de chaque composition

Les valeurs en parfum, conservateur et eau étant alors ajustées comme indiqué dans le tableau 1 ci-dessus.

Les pourcentages en poids d'extraits d'immortelle et d'extrait de Janie rouge sont indiqués dans le tableau 2 suivant.

**Tableau 2: Pourcentage en poids d'extrait huileux d'immortelle, d'extrait aqueux d'immortelle, d'huile essentielle d'immortelle et d'extrait de Janie rouge par rapport au poids total de la composition**

| Composition | % en poids d'extrait huileux d'immortelle par rapport au poids de la composition (%) | % en poids d'extrait aqueux d'immortelle par rapport au poids de la composition (%) | % en poids d'huile essentielle d'immortelle par rapport au poids de la composition (%) | % en poids d'extrait aqueux de Janie rouge par rapport au poids de la composition (%) |
|---|---|---|---|---|
| N°1 | 0,01 | 0,5 | 0,1 | 3 |
| N°2 | 0,02 | 0,01 | 0,05 | 1 |
| N°3 | 0,01 | 0,2 | 0,01 | 2 |
| N°4 | 0,03 | 0,1 | 0,1 | 0,5 |
| N°5 | 0,1 | 0,01 | 0,05 | 3 |
| N°6 | 0,03 | 0,1 | 0,05 | 2 |
| N°7 | 0,01 | 0,01 | 0,2 | 0,1 |
| N°8 | 0,02 | 0,02 | 0,1 | 1 |
| N°9 | 0,03 | 0,01 | 0,05 | 2 |
| N°10 | 0,1 | 0,1 | 0,1 | 3 |
| N°11 | 0,05 | 0,05 | 0,02 | 0,5 |

### Exemple 2: Effet sur l'expression des gènes de la peau d'un extrait de Janie rouge, d'huile essentielle d'immortelle et d'un mélange comprenant un extrait de Janie rouge et d'huile essentielle d'immortelle

Pour l'extrait de Janie rouge, il s'agissait d'un extrait hydroglycérique (50/50 v/v) de *Jania rubens* commercialisé par la société CODIF sous la référence commerciale Actiporine 8G.

Pour l'huile essentielle d'immortelle, il s'agissait d'une huile essentielle obtenue par hydrodistillation et fournie par les sociétés TEPPE ROSSE, STEPHAN FRANCISCI, GAEC de l'ASTRATELLA ou mélange de deux ou trois de ces huiles.

Le transcriptome est l'ensemble des ARN messagers issus de l'expression d'une partie du génome dans un tissu ou un type cellulaire donné. La caractérisation et la quantification du transcriptome permettent d'identifier les gènes régulés dans des conditions particulières, de déterminer les mécanismes de régulation de ces gènes et de définir les réseaux d'expression ou des voies d'activation de ces gènes. Une des techniques utilisées pour mesurer simultanément le niveau d'expression d'un grand nombre d'ARN messagers différents est celle de la puce à ADN. Les puces à ADN permettent de mesurer et de visualiser très rapidement les différences d'expression à l'échelle d'un génome complet.

### 1. Matériel et méthode

L'extrait de Janie rouge, l'huile essentielle d'immortelle ou la combinaison des deux extraits ont été mis en présence d'explants de peau en survie. L'huile essentielle d'immortelle a été solubilisée à 10% dans le Diméthylsulfoxide (DMSO) avant d'être appliquée dans le milieu de culture. L'extrait de Janie rouge a quant à lui été directement dilué dans le milieu de culture.

4 explants de peau provenant de plastie abdominale d'un patient de 62 ans ont été incubés indépendamment. Ils ont été incubés en présence de l'extrait de Janie rouge, de l'huile essentielle d'immortelle ou de leur association pendant 24h dans un milieu « skin culture médium » de la société Biopredic à 37°C à 5% de CO₂ en atmosphère humide (98%) à l'interface air-liquide. 4 explants de peau non traités ou traités avec le solvant DMSO à 1% ont été incubés dans les mêmes conditions.

Le tableau 3 ci-dessous récapitule les concentrations appliquées pour chaque composition.

**Tableau 3 : concentration appliquée pour chaque composition**

| **Composition** | **Pourcentage (%)** |
|---|---|
| Extrait de Janie rouge | 0,2 |
| Huile essentielle d'immortelle | 0,1 |
| Extrait de Janie rouge | 0,2 |
| Et | |
| Huile essentielle d'immortelle | 0,1 |

### Extraction des ARNs totaux

Après incubation, les explants de peau ont été broyés dans le tampon préconisé par le fabricant du RNeasy mini kit (Qiagen, Hilden, Allemagne) puis les ARN totaux ont été extraits en utilisant ce même kit. Les ARN totaux ont été quantifiés puis leur qualité a été vérifiée en utilisant le BlOanalyzer 2100 avec le RNA 6000 Nano LabChip Kit (Agilent Technologies, Santa Clara, USA).

### Mesure de l'expression génique sur puce à oligonucléotides et acquisition des données :

Les puces à oligonucléotides de 60mers sur des lamelles en verre 1"x3", SurePrint G3 Human Gene Expression 8x60K v2 Microarray (G4851B, Agilent Technologies, Santa Clara, USA) ont été utilisées pour l'analyse de l'expression des gènes (SurePrint G3 Human Gene Expression 8x60K v2, Agilent Technologies, Santa Clara, USA).

Une documentation ainsi que le protocole expérimental complet est disponible sur le site d'Agilent Technologies [32].

En bref, la rétrotranscription et l'amplification des ARN totaux en ADNc puis en ARNc et leur marquage avec la cyanine 3 est réalisé à l'aide du Low Input Quick Amp Labeling kit (Agilent Technologies). La purification des ARNc a été effectuée à l'aide du RNeasy mini kit (Qiagen, Hilden, Allemagne) et l'hybridation avec le Microarray hybridization chamber kit (Agilent Technologies, Santa Clara, USA). Les puces ont été ensuite scannées en utilisant le scanner SureScan (Agilent Technologies, Santa Clara, USA) et le logiciel Scan control (Agilent Technologies, Santa Clara, USA). Les images scannées ont ensuite été extraites et normalisées avec le logiciel Feature Extraction (Agilent Technologies, Santa Clara, USA).

L'analyse statistique des données a été réalisée à l'aide du logiciel Genespring GX 13 (Agilent Technologies, Santa Clara, USA).

Le logiciel Ingenuity Pathways Analysis (Ingenuity^{®} Pathway Analysis (IPA), Ingenuity Systems, Redwood City, CA, USA - http://www.ingenuity.com) a été ensuite utilisé pour analyser et prédire l'état d'activation des voies biologiques modulées par l'extrait de Janie rouge, l'huile essentielle d'immortelle ou leur association.

Pour chaque condition de traitement, 4 expériences indépendantes de marquages ont été réalisées (soit 4 puces oligonucléotides par condition, correspondant aux 4 explants de peau traités indépendamment pour chaque condition) pour augmenter la reproductibilité des données.

Les gènes exprimés par les explants de peau traités ont été considérés comme étant induits ou inhibés si leur niveau d'expression différentielle était supérieur à un facteur 2 comparativement à ceux des explants contrôles. Les explants non traités ont servi de contrôle pour la condition traitée par l'extrait de Janie rouge et les explants traités avec le solvant DMSO à 1% ont servi de contrôle pour la condition traitée avec l'huile essentielle d'immortelle ou la combinaison de l'extrait de Janie rouge et l'huile essentielle d'immortelle en combinaison. Un moderated T-test corrigé du taux de faux positifs a été appliqué avec la procédure de Benjamini & Hochberg [33] grâce au logiciel GeneSpring afin de calculer la différence statistique d'expression génique entre les explants non traités ou traités avec le solvant DMSO et les explants traités avec les actifs ou leur association. Les gènes dont la p-value était inférieure ou égale à 0,05 ont été considérés comme différentiellement exprimés.

Ces gènes ont ensuite été analysés par le logiciel Ingenuity Pathways Analysis. Ce logiciel permet une analyse fonctionnelle des gènes régulés dans les différentes conditions de traitement, une analyse des voies de signalisation dans lesquels s'inscrivent ces gènes et l'analyse des régulateurs en amont de ces voies. Un Z-score a été calculé par le logiciel par rapport aux gènes régulés de manière significative. Un Z-score >2 ou <-2 indique dans un intervalle de confiance de 99% que les voies biologiques induites ou réprimées ne le sont pas uniquement par chance, mais bien spécifiquement.

**Tableau 4: Z-score de la voie de la différenciation des cellules épidermiques en réponse au traitement des explants de peau par l'extrait de Janie rouge, l'huile essentielle d'immortelle et leur association**

| | Extrait de Janie rouge | Huile Essentielle (HE) Immortelle | Extrait de Janie rouge et HE Immortelle |
|---|---|---|---|
| Différenciation des cellules épidermiques | - | 1,948 | 2,398 |

Le tableau ci-dessous présente la différence d'expression des gènes qui sont impliqués dans la différenciation des cellules épidermiques suite au traitement des explants de peau avec l'extrait de Janie rouge, l'huile essentielle d'immortelle et leur association.

**Tableau 5 : Différence d'expression en fonction de l'incubation avec un extrait de Janie rouge, une huile essentielle d'immortelle ou une combinaison d'extrait de Janie rouge et une huile essentielle d'immortelle.**

| **GENES** | | **Genbank Accession** | **STATUT dans étude: Expression par rapport au contrôle non traité ou au contrôle solvant** | | |
|---|---|---|---|---|---|
| **Symbole** | **Nom** | | **Janie rouge** | **HE Immortelle** | **Janie rouge et HE Immortelle** |
| AURKB | aurora kinase B | NM_004217 | - | -3,07 | -4,89 |
| CDK1 | cyclin-dependent kinase 1 | NM_001786 | - | -2,40 | -2,92 |
| EGF | epidermal growth factor | NM_001963 | - | - | 2,21 |
| FLG | Filaggrin | NM_002016 | - | - | 2,03 |
| FLG2 | Filaggrin family member 2 | NM_0010143 42 | - | - | 2,24 |
| FOXM1 | forkhead box M1 | NM_202002 | - | -2,75 | -2,79 |
| FOXN1 | forkhead box N1 | NM_003593 | - | - | -2,09 |
| GAK | cyclin G associated kinase | AK131464 | - | - | 2,35 |
| GLI2 | GLI family zinc finger 2 | NM_005270 | - | - | 2,04 |
| HDAC2 | histone deacetylase 2 | NM_001527 | - | 2,05 | 2,28 |
| HGF | hepatocyte growth factor (hepapoietin A; scatter factor) | NM_0010109 34 | - | - | 4,29 |
| HOXA7 | homeobox A7 | NM_006896 | - | - | -2,64 |
| IL20 | interleukin 20 | NM_018724 | - | 2,51 | 2,54 |
| LATS2 | large tumor suppressor kinase 2 | NM_014572 | - | - | 2,11 |
| MAPK8 | mitogen-activated protein kinase 8 | NM_0012785 47 | - | 2,45 | 2,68 |
| PPARD | peroxisome proliferator-activated receptor delta | NM_006238 | - | 2,85 | 3,06 |
| TNF | tumor necrosis factor | NM_000594 | - | - | -3,68 |
| TNFSF10 | tumor necrosis factor (ligand) superfamily, member 10 | NM_003810 | - | -8,80 | -12,02 |
| TNFSF12 | tumor necrosis factor (ligand) superfamily, member 12 | NM_003809 | - | -2,11 | -2,16 |
| TP63 | tumor protein p63 | NM_003722 | - | -3,03 | -3,39 |
| VDR | vitamin D (1,25-dihydroxyvitamin D3) receptor | NM_0010175 35 | - | -2,37 | -2,49 |
| WNT16 | wingless-type MMTV intégration site family, member 16 | NM_057168 | - | -4,21 | -7,57 |

Dans le tableau précité, la valeur chiffrée représente le facteur de stimulation ou d'inhibition par rapport à la peau non traitée, db EST est une base de données regroupant la référence du gène ainsi que sa séquence correspondant au gène spotté sur les puces oligonucléotides de 60mers sur des lamelles en verre 1"×3", SurePrint G3 Human Gene Expression 8x60K v2 Microarray (G4851B, Agilent Technologies, Santa Clara, USA), et «-» l'absence de régulation du gène.

Les résultats obtenus mettent en évidence que le traitement des explants par l'association de l'extrait de Janie rouge et de l'huile essentielle d'immortelle induit une augmentation de la différentiation des cellules épidermique avec un Z-score de 2,398 alors que l'huile essentielle d'Immortelle seule n'induit qu'un Z-score de 1,948 non significatif et que l'extrait de Janie rouge seule n'induit pas cette voie.

Ainsi, tel que démontré ci-dessus, un exemple de composition selon l'invention comprenant un mélange d'au moins un extrait de Janie rouge et d'huile essentielle d'immortelle permet avantageusement, notamment de part une augmentation de la différenciation des cellules épidermiques, de renforcer la fonction barrière de la peau, de maintenir une meilleure hydratation, de stimuler les défenses de la peau et d'améliorer la qualité de surface de la peau.

Tel que démontré dans le tableau, le mélange comprenant l'extrait de Janie rouge et l'huile essentielle d'immortelle permet avantageusement de stimuler l'expression des gènes codants pour la filaggrine et pour la filaggrine 2. Ces 2 protéines ont un rôle primordial dans le maintien l'hydratation cutanée. Elles sont co-localisées dans les granules de keratohyaline des kératinocytes granuleux et dans la matrice cytoplasmique des cornéocytes. Lorsque la filaggrine est dégradée en acides aminés, elle va constituer en partie le facteur d'hydratation naturel de la peau (NMF) [21]. Le rôle exact de la filaggrine 2 n'a pas encore été découvert, mais il semble similaire à celui de la filaggrine et est importante pour le bon déroulement de la cornification et pour la constitution d'un stratum corneum fonctionnel [34].

Le mélange comprenant l'extrait de Janie rouge et l'huile essentielle d'immortelle permet également avantageusement d'inhiber l'expression du gène codant pour HOXA7. La surexpression de ce gène est connue pour diminuer l'induction de la transglutaminase 1 et l'expression de HOXA7 est inversement corrélée à la différentiation des kératinocytes qui est impliqué dans le bon fonctionnement et la formation de la barrière épidermique [35].

De plus, le mélange comprenant l'extrait de Janie rouge et l'huile essentielle d'immortelle permet également avantageusement d'induire le gène codant pour la cyclin G associated kinase (GAK) qui est connu pour être impliquée dans la formation de la barrière épidermique. En effet, les souris délétées du gène GAK spécifiquement au niveau de la peau meurent à la naissance par dessiccation car leur barrière épidermique est défectueuse [36] due à un défaut dans le processus de différenciation tel que l'absence de couches multiples qui forment normalement l'épiderme et le stratum corneum ne se développe pas correctement.

De même, l'inhibition du gène codant pour le facteur tumoral nécrosant alpha (Tumor Necrosis Factor alpha, TNF-α) par le mélange de l'extrait aqueux et de l'huile essentielle d'immortelle va conduire à une fonction barrière renforcée avantageusement par le mélange de ces 2 actifs. En effet, les protéines filaggrine et loricrine sont connues pour être inhibées par le TNF-α. Le mélange de l'extrait de Janie rouge et d'huile essentielle d'immortelle en inhibant avantageusement le TNF-α va permettre l'expression de la loricrine et de la filaggrine et donc un renforcement de la barrière épidermique.

Par ailleurs, les facteurs de croissance épidermique (EGF) et hépatocytaire (HGF) sont induits uniquement en présence du mélange de l'extrait de Janie rouge et de l'huile essentielle d'immortelle. Ces facteurs de croissance sont essentiels à la prolifération des kératinocytes et leur induction va augmenter le renouvellement épidermique. La diminution de la capacité proliférative des kératinocytes participe à l'affinement de l'épiderme au cours du vieillissement. Ainsi le traitement par le mélange des deux extraits va de manière synergique restaurer cette épaisseur [37]. Les autres gènes régulés également par le mélange de l'extrait de Janie rouge et de l'huile essentielle d'immortelle (tableau 5) contribuent aussi à la formation de la barrière épidermique ou au renouvellement épidermique.

Cet exemple démontre clairement par une analyse transcriptomique ex *vivo* par puces à ADN que la composition de l'invention permet de stimuler des gènes impliqués notamment dans la différenciation des cellules épidermiques.

Cet exemple démontre également clairement qu'une composition comprenant un extrait de Janie rouge et un extrait d'immortelle permet avantageusement de stimuler le fonctionnement et/ou la fonction de la barrière épidermique, permettant notamment de protéger la peau des agressions extérieures, de maintenir/augmenter l'hydratation de la peau, de prévenir et/ou traiter le vieillissement de la peau, par exemple en conservant et/ou restaurant l'épaisseur de l'épiderme, d'améliorer la qualité de surface de la peau et donc d'avoir un teint plus éclatant par une meilleure réflexion de la lumière.

### Exemple 3 : Test d'usage d'un exemple de composition cosmétique selon l'invention et évaluation des qualités cosmétiques de la composition

Afin de valider les effets d'un exemple de composition selon l'invention, un test d'usage a été réalisé sur un panel indépendant de volontaires de sexe féminin.

Le test d'usage a été mené sur un panel de 54 volontaires âgées entre 42 à 65 ans (56 ans en moyenne), présentant au niveau de la peau du visage et du cou des rides et ridules, un manque d'éclat, un manque de fermeté de la peau.

Après avoir respiré le parfum, la composition a été appliquée bi-quotidiennement matin et soir pendant 28 jours en petite dose à l'aide d'une spatule, par mouvement lissant de l'intérieur vers l'extérieur du visage et du cou, préalablement nettoyés.

La composition utilisée comprenait les composés indiqués dans le tableau 1 et 0,03% en poids d'extrait huileux d'immortelle, 0,1% en poids d'extrait aqueux d'immortelle, 0,05% en poids d'huile essentielle d'immortelle et 2% en poids d'extrait aqueux de Janie rouge par rapport au poids total de la composition.

**Tableau 6 : Jugement global de la composition**

| Appréciation générale du produit | |
|---|---|
| **A l'application** | |
| La texture est soyeuse | 93% |
| La texture évolue au cours de l'application | 76% |
| Le produit fond sur la peau | 78% |

| **Immédiatement après la première application** | |
|---|---|
| La peau est nourrie | 94% |
| La peau est réconfortée | 93% |
| La peau est souple | 91% |
| La peau semble défroissée | 70% |
| La peau est plus tonique | 70% |

| **Après 28 jours d'utilisation** | |
|---|---|
| La peau est intensément nourrie | 83% |
| Le produit offre une vraie sensation de confort | 83% |
| La peau est plus souple | 81% |
| Les traits semblent reposés | 78% |
| La peau est plus ferme | 61% |
| La peau est plus élastique | 69% |

Tel que démontré dans le tableau 6, un exemple de composition selon l'invention comprenant 0,03% en poids d'extrait huileux d'immortelle, 0,1% en poids d'extrait aqueux d'immortelle, 0,05% en poids d'huile essentielle d'immortelle et 2% en poids d'extrait aqueux de Janie rouge par rapport au poids total de la composition a été donc considéré comme efficace. En particulier, tel que démontré, un exemple de composition selon l'invention permet de traiter les signes du vieillissement, notamment une amélioration de l'hydratation de la peau, le traitement des rides et ridules, l'amélioration de l'élasticité et de la fermeté de la peau. Tel que démontré, la composition permet avantageusement un traitement immédiatement après application et dans le temps.

### Exemple 4: Test d'usage d'un exemple de composition cosmétique selon l'invention et évaluation des qualités cosmétiques de la composition

Le test d'usage a été mené sur un panel de 35 volontaires féminins de type asiatique entre 40 à 65 ans (54,6 ans en moyenne) représentant tous les types de peau, par exemple sèche, normale, mixte et grasse. Elles avaient une peau terne, un teint clair avec présence de tâches pigmentaires, à savoir avec une densité définie selon l'Aging Atlas de grade ≥2, et au moins une tâche bien définie au niveau de la joue, par exemple d'un diamètre de 2,5 à 3 mm.

Après avoir respiré le parfum, la composition a été appliquée bi-quotidiennement matin et soir jusqu'à 12 semaines en petite dose à l'aide d'une spatule, par mouvement lissant de l'intérieur vers l'extérieur du visage et du cou, préalablement nettoyés.

La composition utilisée comprenait les composés indiqués dans le tableau 1 et 0,03% en poids d'extrait huileux d'immortelle, 0,1% en poids d'extrait aqueux d'immortelle, 0,08% en poids d'huile essentielle d'immortelle et 2% en poids d'extrait aqueux de Janie rouge par rapport au poids total de la composition.

**Tableau 7 : Jugement global de la composition**

| **Immédiatement après la première application** | |
|---|---|
| Le teint est illuminé | 80% |
| La peau est radiante | 89% |
| La peau est souple | 94% |

| **Après 8 semaines d'utilisation** | |
|---|---|
| Le teint est visiblement éclairci | 94% |
| La peau semble lumineuse | 91% |
| L'apparence des irrégularités pigmentaires, tel que les points noirs, est réduite | 86% |
| La taille des défauts pigmentaires est réduite | 89% |
| Le teint est unifié | 86% |
| Le produit réduit l'apparence des différences de teint | 77% |
| la peau est débarrassée de son voile terne | 83% |
| Les capacités de la peau à capter et refléter la lumière sont augmentées | 83% |
| La peau révèle sa clarté | 80% |
| La peau retrouve sa translucidité naturelle | 94% |
| La peau retrouve sa lumière originelle | 94% |
| La peau est plus lisse | 100% |
| La texture de la peau semble raffinée | 100% |
| Les irrégularités de la texture de la peau sont réduites | 89% |
| La qualité de la peau est améliorée | 97% |
| L'uniformité de la peau est améliorée | 100% |
| La peau apparait plus juste | 94% |
| La peau brille avec un éclat de jeunesse | 83% |
| La peau apparait sereine | 86% |
| Le visage s'illumine | 94% |
| La peau apparait plus belle | 94% |

| **Après 12 semaines d'utilisation** | |
|---|---|
| Le teint est visiblement éclairci | 91% |
| L'apparence des irrégularités pigmentaires, tel que les points noirs, est réduite | 80% |
| La taille des défauts pigmentaires est réduite | 89% |
| Le teint est unifié | 80% |
| Les capacités de la peau à capter et refléter la lumière sont augmentées | 91% |
| La peau révèle sa clarté | 89% |
| La peau retrouve sa translucidité naturelle | 94% |
| La peau retrouve sa lumière originelle | 91% |
| La texture de la peau semble raffinée | 94% |
| Les irrégularités de la texture de la peau sont réduites | 94% |
| La qualité de la peau est améliorée | 94% |
| La peau apparait plus claire | 91% |
| La peau brille avec un éclat de jeunesse | 89% |
| Le teint est harmonieux | 89% |
| La peau apparait sans défaut | 69% |
| Le teint est visiblement éclaircit | 89% |
| La peau apparait plus belle | 91% |

Tel que démontré dans le tableau 7, un exemple de composition selon l'invention comprenant 0,03% en poids d'extrait huileux d'immortelle, 0,1% en poids d'extrait aqueux d'immortelle, 0,08% en poids d'huile essentielle d'immortelle et 2% en poids d'extrait aqueux de Janie rouge par rapport au poids total de la composition a été donc considéré comme efficace. En particulier, tel que démontré, un exemple de composition selon l'invention permet de traiter les signes du vieillissement des peaux asiatiques, notamment une amélioration de l'éclat du teint, de son homogénéité, de sa luminosité et/ou de réduire les défauts pigmentaires de la peau. En outre, tel que démontré un exemple de composition selon l'invention permet d'améliorer l'aspect de la peau avec notamment une texture plus uniforme et plus lisse. Tel que démontré, la composition permet avantageusement un traitement immédiatement après application et dans le temps.

En outre, une étude visuelle de la densité des taches pigmentaires et de l'éclat du teint en utilisant le score C.L.B.T. tel que décrit dans Musnier et al. Visual evaluation in vivo of 'complexion radiance' using the C.L.B.T. sensory methodology. Skin Res Technol. 2004 Feb;10(1):50-6 [38] a été effectuée. Chaque paramètre a été mesuré après 2 et 3 mois d'application de la composition et comparé aux mesures effectuées avant la première application de la composition. Ainsi, une évaluation de la peau au niveau du visage par 3 juges entraînés des 35 volontaires précitées, a été effectuée.

Les descripteurs de couleurs, à savoir Olive, Jaune ou Rose et d'aspects physiques, à savoir la luminosité, brillance et transparence, ont été préalablement définis. En particulier, une augmentation de la couleur rose signifiait un effet aspect plus sain, une diminution de la couleur «jaune» un bon effet (teint moins jaunâtre), une diminution de la couleur «olive» signifiait un bon effet et un effet aspect plus sain (le teint moins olive-verdâtre), une augmentation de la luminosité: la peau reflète la lumière d'une manière légèrement plus intense, une augmentation de la luminosité: le teint a un aspect en termes de texture et de couleur, une augmentation de la transparence: la peau est plus mince.

L'évaluation a été réalisée en chambre noire sous température et atmosphère contrôlée (22 +/- 2°C, 50 +/- 10% d'hygrométrie). Après 20 minutes d'acclimatation dans la pièce contrôlée, les sujets ont été placés entre 2 lampes diffusant une lumière dite « de jour ».

Le teint a été évalué individuellement par 3 juges entraînés, sans connaissance sur les données obtenues au temps précédent. Les 3 juges n'étaient pas autorisés à communiquer entre eux. L'évaluation des 3 couleurs a été réalisée à l'aide d'une échelle visuelle des 3 principales couleurs de la peau du visage (olive/jaune/rose). Un grade a été attribué pour chaque couleur représentant un pourcentage de saturation. Chaque juge a attribué un grade pour chaque couleur à T0, c'est-à-dire avant traitement.

Une évaluation Clinique de la luminosité, la brillance et la transparence (LBT) a été réalisée par 3 juges entraînés. Cette évaluation a été retranscrite sur une échelle analogique allant de 0 (pas de luminosité/brillance/transparence) à 100 (luminosité/brillance/transparence maximale).

La moyenne des scores attribués par les 3 juges a été réalisée pour chacun des descripteurs.

Pour les descripteurs de couleur, les résultats ont été exprimés en pourcentage de saturation de la couleur.

Pour les descripteurs LBT, les résultats ont été retranscris par un score allant de 0 à 10.

L'interprétation des résultats de C.L.B.T. a été effectuée sur la base des variations significatives des descripteurs pris séparément:
Analyse statistique des données: La normalité de distribution a été vérifiée à l'aide du test Shapiro-Wilk test (seuil à 1%). L'analyse statistique de l'évolution des paramètres mesurés a été effectuée à l'aide du test t de Student bilatéral apparié (dans le cas d'une distribution normale) ou le test de Wilcoxon (distribution non normale). Le seuil de significativité a été fixé à 5%.

Le tableau 8 ci-dessous résume les résultats obtenus

| Paramètres | Après 2 mois | Après 3 mois |
|---|---|---|
| Densité des tâches | - 23% | -19% |
| Uniformité du teint | +17% | +16% |

| Scorage CLBT | | |
|---|---|---|
| Couleur olive | Ns | Ns |
| Couleur jaune | - 3,9% | -6% |
| Couleur rose | Ns | Ns |
| Luminosité | Ns | Ns |
| Clarté | +3,7 | +6,1 |
| Transparence | Ns | Ns |

Tel que démontré dans le tableau 8 ci-dessous, un exemple de composition selon l'invention permet avantageusement de réduire la densité des taches pigmentaires et améliore l'uniformité du teint. La composition éclaircit également le teint (+6,1% à 3 mois) et en diminue la teinte jaune (-6% à 3 mois). Cet exemple démontre donc clairement qu'un exemple de composition selon l'invention est utile et efficace dans un traitement cosmétique du vieillissement cutané.

Tel que démontré dans cet exemple les compositions selon l'invention sont efficaces avec différent phototype de peau, par exemple des phototypes asiatiques.

### Références

1. Koblenzer CS. Psychologic aspects of aging and the skin. Clinics Dermatol 1996;14:171-7
2. West MD. The cellular and molecular biology of skin aging. Arch dermatol 1994;130:87-95
3. Grove GL, Kligman AM. Age-associated changes in human epidermal cell renewal. J Gerontol 1983;38:137-42
4. Watt FM. Involucrin and other markers of keratinocyte terminal differentiation. J Invest Dermatol. 1983;81:100s-3s
5. Mehrel T, Hohl D, Rothnagel JA, Longley MA, Bundman D, Cheng C, Lichti U, Bisher ME, Steven AC, Steinert PM, et al. Identification of a major keratinocyte cell envelope protein, loricrin. Cell. 1990;61:1103-12
6. Candi E, Schmidt R, Melino G. The cornified envelope: a model of cell death in the skin. Nat Rev Mol Cell Biol. 2005;6:328-40
7. Watt FM, Green H. Involucrin synthesis is correlated with cell size in human epidermal cultures. J. Cell Biol. 1981;90:738-742.
8. Ruhrberg C, Hajibagheri MA, Parry DA, Watt FM. Periplakin, a novel component of cornified envelopes and desmosomes that belongs to the plakin family and forms complexes with envoplakin. J. Cell Biol. 1997;139:1835-1849.
9. Ruhrberg C, Hajibagheri MA, Simon M, Dooley TP, Watt FM. Envoplakin, a novel precursor of the cornified envelope that has homology to desmoplakin. J. Cell Biol. 1996;134:715-729.
10. DiColandrea T, Karashima T, Maatta A, Watt FM. Subcellular distribution of envoplakin and periplakin: insights into their role as precursors of the epidermal cornified envelope. J. Cell Biol. 2000;151:573-586.
11.Sevilla LM, Nachat R, Groot KR, Klement JF, Uitto J, Djian P, Maatta A, Watt FM,. Mice deficient in involucrin, envoplakin, and periplakin have a defective epidermal barrier. J. Cell Biol. 2007;179:1599-1612.
12. Kalinin A, Marekov LN, Steinert PM. Assembly of the epidermal cornified cell envelope. J. Cell Sci. 2001;114:3069-3070.
13. Eckert RL, Sturniolo MT, Broome AM, Ruse M, Rorke EA. Transglutaminase function in epidermis. J. Invest. Dermatol. 2005;124:481-492.
14.Steinert PM, Marekov LN. Initiation of assembly of the cell envelope barrier structure of stratified squamous epithelia. Mol. Biol. Cell 1999;10:4247-4261.
15.Ahvazi B, Boeshans KM, Idler W, Baxa U, Steinert PM. Roles of calcium ions in the activation and activity of the transglutaminase 3 enzyme. J. Biol. Chem. 2003;278:23834-23841.
16. Jackson B, Tilli CM, Hardman MJ, Avilion AA, MacLeod MC, Ashcroft GS, Byrne C. Late cornified envelope family in differentiating epithelia-response to calcium and ultraviolet irradiation. J. Invest. Dermatol. 2005;124:1062-1070.
17. Marshall D, Hardman MJ, Nield KM, Byrne C. Differentially expressed late constituents of the epidermal cornified envelope. Proc. Natl. Acad. Sci. U. S. A. 2001;98:13031-13036.
18. Denda M, Tomitaka A, Akamatsu H, Matsunaga K. Altered distribution of calcium in facial epidermis of aged adults. J. Invest. Dermatol. 2003;121:1557-1558.
19.Chavanas S, Méchin MC, Nachat R, Adoue V, Coudane F, Serre G, Simon M. Peptidylarginine deiminases and deimination in biology and pathology: relevance to skin homeostasis. J Dermatol Sci. 2006;44:63-72.
20. Denecker G, Ovaere P, Vandenabeele P, Declercq W. Caspase-14 reveals its secrets. J Cell Biol. 2008;180:451-8.
21. Brown SJ, McLean WH. One remarkable molecule: filaggrin. J Invest Dermatol. 2012;132:751-62.
22.Steinert PM, Cantieri JS, Teller DC, Lonsdale-Eccles JD, Dale BA. Characterization of a class of cationic proteins that specifically interact with intermediate filaments. Proc Natl Acad Sci U S A. 1981 Jul;78(7):4097-101
23. Rawlings AV, Harding CR. Moisturization and skin barrier function. Dermatol Ther. 2004;17 Suppl 1:43-8
24. Elias PM, Steinhoff M. "Outside-to-inside" (and now back to "outside") pathogenic mechanisms in atopic dermatitis. J Invest Dermatol. 2008 May;128(5):1067-70
25. FR3000389
26. FR0111224
27. FR0605953
28. FR0905904
29. FR1153960
30. Farid Chemat : Eco-extraction du végétal, Editions Dunod, 2011
31. BENAISSI, K. Le CO2 supercritique appliqué à l'extraction végétale. Techniques de l'ingénieur Développement de solvants alternatifs et intensification des procédés. 2013. http://www.techniques-ingenieur.fr/
32. http://www.chem.agilent.com/library/usermanuals/Public/G4140-90040_GeneExpression_ OneColor_6.6.pdf
33. Benjamini Y & Hochberg Y. Controlling the false discovery rate : a practical and powerful approach to multiple testing. Journal of the Royal Statistical Society B. 1995;57:289-300.
34. Wu Z, Hansmann B, Meyer-Hoffert U, Gläser R, Schröder JM. Molecular identification and expression analysis of filaggrin-2, a member of the S100 fused-type protein family. PLoS One. 2009;4:e5227
35. La Celle PT, Polakowska RR. Human homeobox HOXA7 regulates keratinocyte transglutaminase type 1 and inhibits differentiation. J Biol Chem. 2001;276:32844-53
36.Segre JA, Bauer C, Fuchs E. Klf4 is a transcription factor required for establishing the barrier function of the skin. Nat Genet. 1999;22:356-60
37.Sato C, Tsuboi R, Shi CM, Rubin JS, Ogawa H. Comparative study of hepatocyte growth factor/scatter factor and keratinocyte growth factor effects on human keratinocytes. J Invest Dermatol. 1995;104:958-63
38. Musnier C, Piquemal P, Beau P, Pittet JC. Visual evaluation in vivo of 'complexion radiance' using the C.L.B.T. sensory methodology.Skin Res Technol. 2004 Feb;10(1):50-6.

## Revendications

1. Composition cosmétique comprenant au moins un extrait de Janie rouge, une huile essentielle d'immortelle et un extrait aqueux d'immortelle et/ou un extrait huileux d'immortelle, dans laquelle la concentration d'extrait de Janie rouge est de 0,001% à 5% en poids par rapport au poids total de la composition.

2. Composition selon la revendication 1, dans laquelle ledit extrait de Janie rouge est choisi dans le groupe comprenant un extrait hydrosoluble de Janie rouge, un extrait liposoluble de Janie rouge ou un mélange de ceux-ci.

3. Composition selon l'une quelconque des revendications précédentes dans laquelle la concentration d'extrait d'immortelle est de 0,001 à 10 % en poids par rapport au poids total de la composition.

4. Composition selon l'une quelconque des revendications précédentes dans laquelle l'extrait d'immortelle est un extrait d'*Helichrysum Italicum.*

5. Composition selon l'une quelconque des revendications précédentes dans laquelle l'extrait de Janie rouge est un extrait de *Jania rubens.*

6. Composition selon l'une quelconque des revendications précédentes dans laquelle la composition est sous une forme choisie parmi une crème, un sérum, un lait, un gel, une lotion, un onguent, une huile, un baume, un masque.

7. Utilisation cosmétique d'une composition selon l'une quelconque des revendications 1 à 6 dans un traitement cosmétique non thérapeutique anti-âge.

8. Utilisation cosmétique d'une composition selon l'une quelconque des revendications 1 à 6 dans un traitement cosmétique non thérapeutique traitement du vieillissement cutané, des rides et ridules, de la perte de fermeté cutanée, de la perte d'élasticité cutanée et des peaux sèches

9. Composition cosmétique selon l'une quelconque des revendications 1 à 6 pour son utilisation dans un traitement pour la protection de la peau des agressions extérieures.

10. Procédé de traitement cosmétique non thérapeutique comprenant l'application sur la peau d'une composition cosmétique selon l'une quelconque des revendications revendication 1 à 6.

11. Trousse de soin cosmétique pour la mise en œuvre du procédé selon la revendication 10 comprenant une composition selon l'une quelconque des revendications 1 à 6 et une notice d'utilisation.

## Patentansprüche

1. Kosmetische Zusammensetzung, umfassend mindestens einen Extrakt des Schlankwurzel-Korallengrases, ein ätherisches Öl des Immerwurzelkrauts und einen wässrigen Extrakt des Immerwurzelkrauts und/oder einen öligen Extrakt des Immerwurzelkrauts, wobei die Konzentration des Extrakts des Schlankwurzel-Korallengrases von 0,001 bis 5 Gew.-%, bezogen auf das Gesamtgewicht der Zusammensetzung, beträgt.

2. Zusammensetzung nach Anspruch 1, wobei der Extrakt des Schlankkorallengrases aus der Gruppe ausgewählt ist, die einen wasserlöslichen Extrakt des Schlankkorallengrases, einen fettlöslichen Extrakt des Schlankkorallengrases oder eine Mischung davon umfasst.

3. Zusammensetzung nach einem der vorhergehenden Ansprüche, wobei die Konzentration des Extrakts des Immerwährenden von 0,001 bis 10 Gew.-%, bezogen auf das Gesamtgewicht der Zusammensetzung, beträgt.

4. Zusammensetzung nach einem der vorhergehenden Ansprüche, wobei der Extrakt des Immerwährenden ein Extrakt aus *Helichrysum Italicum* ist.

5. Zusammensetzung nach einem der vorhergehenden Ansprüche, wobei der Extrakt aus dem Schlankkorallen-Kraut ein Extrakt aus *Jania rubens* ist.

6. Die Zusammensetzung nach einem der vorhergehenden Ansprüche, wobei die Zusammensetzung in einer Form vorliegt, die ausgewählt ist aus einer Creme, einem Serum, einer Milch, einem Gel, einer Lotion, einer Salbe, einem Öl, einem Balsam und einer Maske.

7. Kosmetische Verwendung einer Zusammensetzung nach einem der Ansprüche 1 bis 6 in einer nichttherapeutischen kosmetischen Anti-Aging-Behandlung.

8. Kosmetische Verwendung einer Zusammensetzung nach einem der Ansprüche 1 bis 6 in einer nichttherapeutischen kosmetischen Behandlung zur Behandlung von Hautalterung, Falten und feinen Linien, Verlust der Hautfestigkeit, Verlust der Hautelastizität und trockener Haut.

9. Kosmetische Zusammensetzung nach einem der Ansprüche 1 bis 6 zur Verwendung bei einer Behandlung zum Schutz der Haut gegen äußere Einflüsse.

10. Nichttherapeutisches kosmetisches Behandlungsverfahren, umfassend das Auftragen einer kosmetischen Zusammensetzung nach einem der Ansprüche 1 bis 6 auf die Haut.

11. Kosmetisches Pflegeset zur Durchführung des Verfahrens Anspruch 10, umfassend eine Zusammensetzung nach einem der Ansprüche 1 bis 6 und einen Hinweis zur Verwendung.

## Claims

1. A cosmetic composition comprising at least one extract of slender-beaded coral weed, an essential oil of everlasting and an aqueous extract of everlasting and/or an oily extract of everlasting, wherein the concentration of extract of slender-beaded coral weed is from 0.001% to 5% by weight relative to the total weight of the composition.

2. The composition according to claim 1, wherein said extract of slender-beaded coral weed is chosen from the group comprising a watersoluble extract of slender-beaded coral weed, a liposoluble extract of slender-beaded coral weed or a mixture thereof.

3. The composition according to any one of the preceding claims, wherein the concentration of extract of everlasting is from 0.001% to 10% by weight relative to the total weight of the composition.

4. The composition according to any one of the preceding claims wherein the extract of everlasting is an extract of *Helichrysum Italicum.*

5. The composition according to any one of the preceding claims, wherein the extract of slender-beaded coral weed is an extract of *Jania rubens.*

6. The composition according to any one of the preceding claims, wherein the composition is in a form selected from a cream, a serum, a milk, a gel, a lotion, an ointment, an oil, a balm and a mask.

7. The cosmetic use of a composition according to any one of claims 1 to 6 in a nontherapeutic antiaging cosmetic treatment.

8. The cosmetic use of a composition according to any one of claims 1 to 6 in a nontherapeutic cosmetic treatment treating aging of the skin, wrinkles and fine lines, loss of skin firmness, loss of skin elasticity and dry skin.

9. The cosmetic composition according to any one of claims 1 to 6 for use in a treatment for protecting the skin against external attack.

10. A nontherapeutic cosmetic treatment process comprising the application onto the skin of a cosmetic composition according to any one of claims 1 to 6.

11. A cosmetic care kit for performing the process according to claim 10, comprising a composition according to any one of claims 1 to 6 and a notice for use.
